# EUROPEAN PATENT APPLICATION

(11) **EP 4 703 409 A1**
(43) Date of publication of application: **04.03.2026**
(21) Application number: 24797129.4
(22) Date of filing: 25.04.2024
(51) Int. Cl.: C08G 85/00, A61K 31/65, A61K 31/663, A61K 35/28, A61K 35/32, A61K 35/545, A61K 41/00, A61K 47/54, A61K 47/60, A61L 27/18, A61L 27/38, A61L 27/54, A61P 1/02, A61P 17/02, A61P 19/10, C07J 9/00, C08F 16/06, C08G 65/48

(54) **LIGHT-RESPONSIVE CELL DESIGNER MOLECULE**

(30) Priority: 26.04.2023 JP 2023072092
(71) Applicant: The University of Osaka, Osaka 565-0871 (JP)
(72) Inventor: MATSUSAKI, Michiya, Suita-shi, Osaka 565-0871 (JP); NAKAMOTO, Masahiko, Suita-shi, Osaka 565-0871 (JP); ISHII, Masaru, Suita-shi, Osaka 565-0871 (JP); KIKUTA, Junichi, Suita-shi, Osaka 565-0871 (JP); MORIMOTO, Akito, Suita-shi, Osaka 565-0871 (JP)
(74) Representative: Hoffmann Eitle
(86) International application number: PCT/JP2024/016272
(87) International publication number: WO 2024/225384

(57) **Abstract**

The present invention provides a compound which is capable of selectively delivering a cell to a disease site such as a bone or a tooth. The compound comprises a hydrophilic polymer or a derivative thereof, a bile acid or a derivative thereof, and a hydroxyapatite-directed substance which has a photolytic protective group. Also provided is a cell which has adsorbed the compound.

## Description

### Technical Field

The present invention relates to compounds which direct cells to a bone disease site in response to light irradiation, and relates to cells which have adsorbed the compounds.

### Background Art

A technology which imparts osteoporosis recognition function to cells through membrane modification is attracting attention as a cell therapy method for various bone diseases (osteoporosis, heterotopic ossification, calcification) (Non Patent Literature 1-2). On the other hand, due to the widespread distribution of bone tissue throughout the body, there has been a problem with achieving disease site-selective cell delivery, resulting in insufficient therapeutic effects.

### Citation List

### Non Patent Literature

Non Patent Literature 1:
   W. Jin et al., Nat Commun. 2021, 12, 6327
Non Patent Literature 2:
   S. D'Souza et al., Biomaterials 35 (2014) 9447-9458

### Summary of Invention

### Technical Problem

There has been a demand for means to deliver functional cells in a space-time controlled manner to desired disease sites such as bones.

### Solution to Problem

The present inventors have conducted diligent research to solve the above problem and discovered that compounds comprising a hydrophilic polymer or a derivative thereof, a bile acid or a derivative thereof, and a hydroxyapatite-directed substance having a photodegradable protective group can address the issue, thereby completing the present invention.

More specifically, the present invention provides the followings:
(1) A compound comprising a hydrophilic polymer or a derivative thereof, a bile acid or a derivative thereof, and a hydroxyapatite-directed substance having a photodegradable protective group;
(2) The compound according to (1), wherein the hydrophilic polymer is selected from the group consisting of polyethylene glycol, polyvinyl alcohol, polyglutamic acid, polyaspartic acid, polylysine, and polyhistidine;
(3) The compound according to (2), wherein the hydrophilic polymer is polyethylene glycol or polyvinyl alcohol;
(4) The compound according to any one of (1) to (3), wherein the bile acid is selected from the group consisting of cholic acid, chenodeoxycholic acid, deoxycholic acid, lithocholic acid, ursodeoxycholic acid, hyodeoxycholic acid, glycocholic acid, and taurocholic acid;
(5) The compound according to (4), wherein the bile acid is deoxycholic acid or cholic acid;
(6) The compound according to any one of (1) to (5), wherein the hydroxyapatite-directed substance is selected from the group consisting of bisphosphonates, tetracycline, and hydroxyapatite-binding peptides;
(7) The compound according to (6), wherein the hydroxyapatite-directed substance is a bisphosphonate;
(8) The compound according to (7), wherein the bisphosphonate is selected from the group consisting of etidronic acid, alendronic acid, risedronic acid, minodronic acid, ibandronic acid, and zoledronic acid;
(9) The compound according to any one of (1) to (8), wherein the photodegradable protective group is selected from the group consisting of 7-(Diethylamino)coumarin, 7-(Diethylamino)-4-(hydroxymethyl)coumarin, 6-bromo-7-hydroxy-4-(hydroxymethyl)coumarin, 4-(bromomethyl)-7-(diethylamino)coumarin, 1-(2-nitrophenyl)ethanol, 2-(2-nitrophenyl)propan-1-ol, 4',5'-dimethoxy-2'-nitroacetophenone, 1-(6-nitro-1,3-benzodioxole-5-yl)ethanol, 2-nitrobenzyl derivatives, allyl methyl derivatives, allyl carbonyl methyl derivatives, BODIPY derivatives, and Ru complex derivatives;
(10) A method for producing cells directing to hydroxyapatite in response to light irradiation, comprising adsorbing the compound according to any one of (1) to (9) on cells;
(11) An agent for imparting to cells directionality to hydroxyapatite in response to light irradiation, comprising the compound according to any one of (1) to (9);
(12) A kit for imparting to cells directionality to hydroxyapatite in response to light irradiation, comprising the compound according to any one of (1) to (9);
(13) Cells on which the compound according to any one of (1) to (9) is adsorbed;
(14) Cells according to (13), wherein they are mesenchymal stem cells, induced pluripotent stem cells, osteoblasts, osteoclasts, osteoprogenitor cells, or osteoclast precursor cells; and
(15) A compound comprising a hydrophilic polymer or a derivative thereof, a bile acid or a derivative thereof, and a hydroxyapatite-directed substance.

### Advantageous Effects of Invention

According to the present invention, it is possible to deliver functional cells to a desired disease site such as bone at a desired timing.

### Brief Description of Drawings

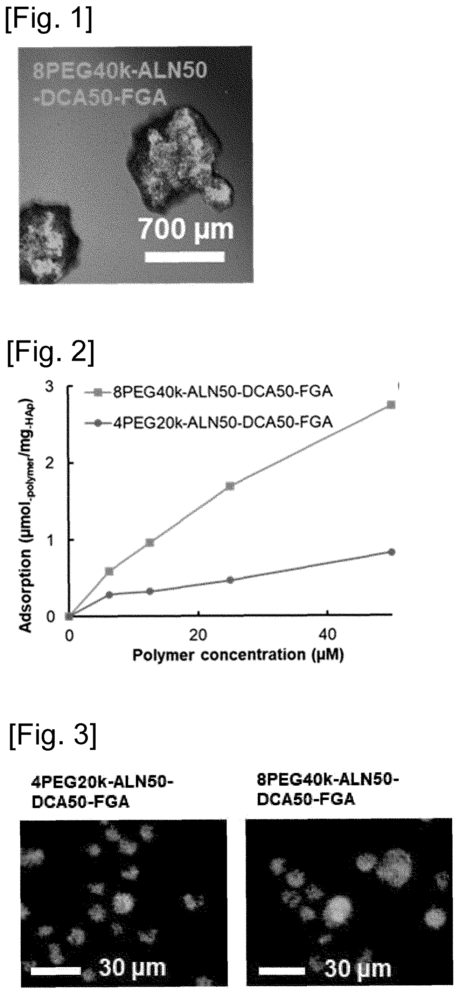

[Figure 1] Figure 1 is a microscopic photograph showing the adsorption of 8PEG40K-ALN50-DCA50-FGA being adsorbed on hydroxyapatite.
[Figure 2] Figure 2 is a graph showing the adsorption amount on hydroxyapatite of 8PEG40K-ALN50-DCA50-FGA (square mark) and 4PEG20K-ALN50-DCA50-FGA (circle mark). The horizontal axis represents the concentration of the inventive compounds (µM), and the vertical axis represents the amount of the inventive compounds per 1 mg of hydroxyapatite (µmol).
[Figure 3] Figure 3 is a microscopic photograph showing the adsorption of 8PEG40K-ALN50-DCA50-FGA (right panel) or 4PEG20K-ALN50-DCA50-FGA (left panel) being adsorbed on normal human skin fibroblasts.

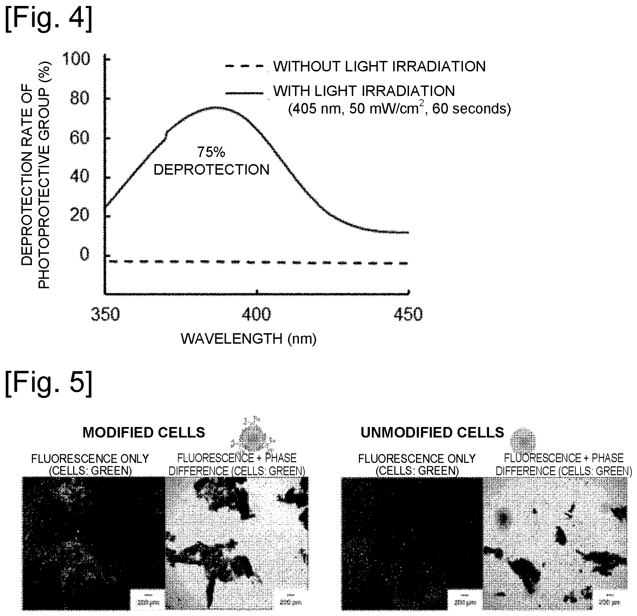

[Figure 4] Figure 4 is a graph (lower panel) showing the deprotection rate when light is irradiated on 4PEG20K-ALN100-DEACM (where alendronic acid is protected with 7-(diethylamino)coumarin (DEACM)). The horizontal axis represents the wavelength of light (nm), and the vertical axis represents the deprotection rate of the photoprotective group (%).
[Figure 5] The left panel of Figure 5 is a microscopic photograph showing the accumulation of mesenchymal stem cells adsorbed with 4PEG20K-ALN50-DCA50-FGA onto mouse bone. The right panel of Figure 5 shows a microscopic photograph showing the accumulation of mesenchymal stem cells not adsorbed with 4PEG20K-ALN50-DCA50-FGA onto mouse bone. Unmodified cells refer to mesenchymal stem cells which have not adsorbed 4PEG20K-ALN50-DCA50-FGA, while modified cells refer to mesenchymal stem cells which have adsorbed 4PEG20K-ALN50-DCA50-FGA.

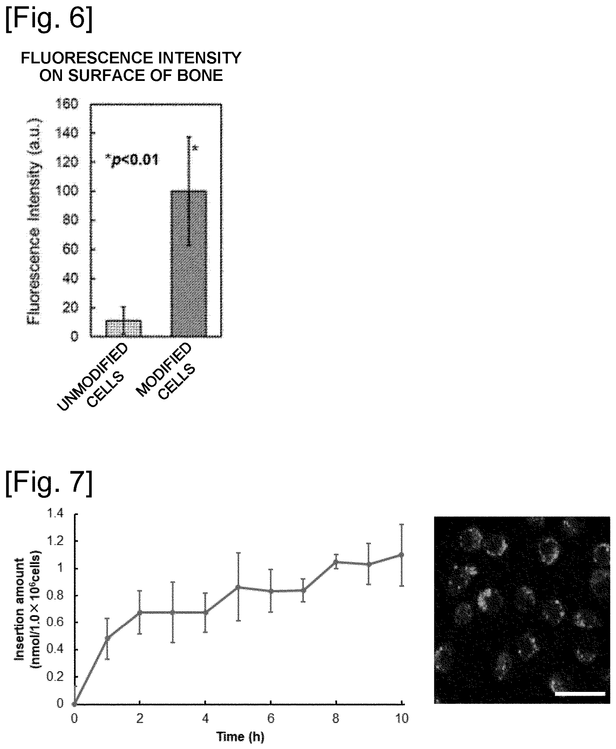

[Figure 6] Figure 6 is a graph comparing the accumulation of mesenchymal stem cells which have or have not adsorbed 4PEG20K-ALN50-DCA50-FGA onto mouse bone by quantifying fluorescence intensity. Unmodified cells refer to mesenchymal stem cells which have not adsorbed 4PEG20K-ALN50-DCA50-FGA, while modified cells refer to mesenchymal stem cells which have adsorbed 4PEG20K-ALN50-DCA50-FGA.
[Figure 7] The left panel of Figure 7 is a graph showing the investigation results of the amount of 8PEG8PEG40k-ALN50-DCA50-FGA adsorbed on osteoclasts over time. The right panel of Figure 7 shows a confocal fluorescence microscopic image depicting the adsorption of 8PEG40k-ALN50-DCA50-FGA on osteoclast precursor cells.

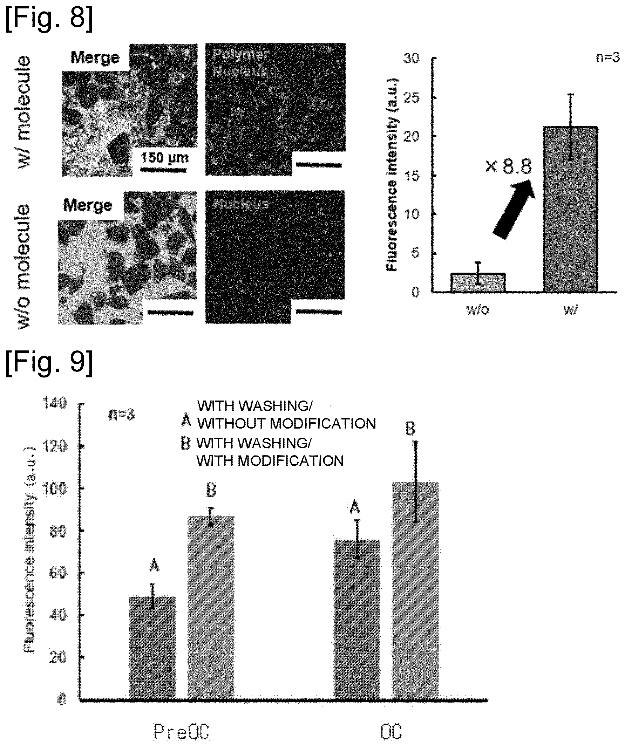

[Figure 8] The upper panels in the left of Figure 8 show the results of adding 8PEG40k-ALN50-DCA50-FGA-adsorbed osteoclast precursor cells to hydroxyapatite, while the lower panels show the results of adding non-adsorbed cells to hydroxyapatite. w/molecule indicates that osteoclast precursor cells adsorbed with 8PEG40k-ALN50-DCA50-FGA were used, while w/o molecule indicates that osteoclast precursor cells not adsorbed with 8PEG40k-ALN50-DCA50-FGA were used. The right panel of Figure 8 shows the results of analyzing and quantifying the blue fluorescent intensity of the microscopic image using ImageJ.
[Figure 9] Figure 9 is a graph showing the investigation results of the osteolytic action of osteoclast precursor cells (PreOC) adsorbed with 8PEG40k-ALN50-DCA50-FGA, as well as the osteolytic action of osteoclasts (OC) adsorbed with 8PEG40k-ALN50-DCA50-FGA.

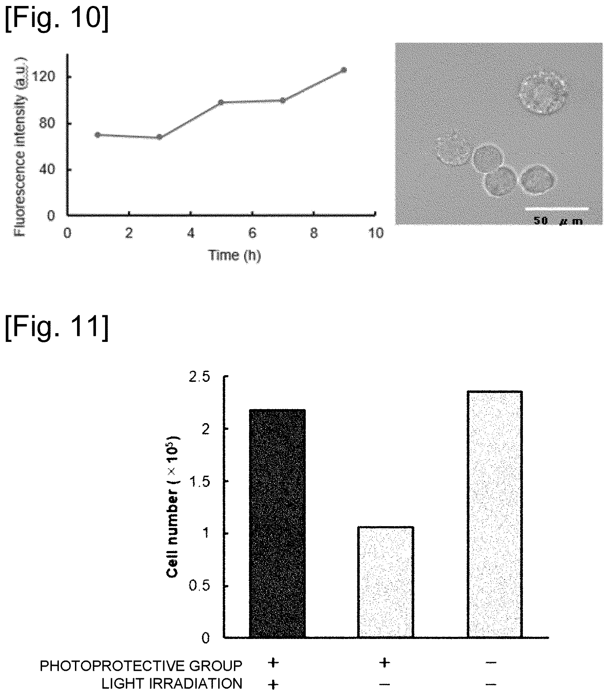

[Figure 10] The left panel of Figure 10 is a graph showing the measurement results of the amount of 8PEG40k-ALN50-DCA50-FGA adsorbed on osteoclasts over time. The right panel of Figure 10 shows a confocal fluorescence microscopic image depicting the adsorption of 8PEG40k-ALN50-DCA50-FGA on osteoclasts after a 9-hour incubation.
[Figure 11] Figure 11 is a graph showing the results of counting the number of cells adsorbed on hydroxyapatite under conditions with and without light irradiation.

### Description of Embodiments

In one aspect, the present invention provides a compound comprising a hydrophilic polymer or a derivative thereof, a bile acid or a derivative thereof, and a hydroxyapatite-directed substance having photodegradable protective group. As used herein, the above compound can be referred to as "the inventive compound ", "the designer molecule of the present invention", or "designer molecule".

In the inventive compounds, hydrophilic polymers or derivatives thereof serve as biocompatible components. Bile acids or derivatives thereof help the adsorption of the inventive compounds on cells. Hydroxyapatite-directed substances help bring close or attach cells, which are adsorbed with the inventive compounds, to biological tissues or biological sites containing hydroxyapatite. Photodegradable protective groups help control the functional expression of the inventive compounds. When the photodegradable protective group is degraded by light irradiation, the functions of the hydroxyapatite-directed substance are exhibited, causing the cells adsorbed with the inventive compounds to be directed towards hydroxyapatite.

A hydrophilic polymer is a polymer which contains charged or polar functional groups and is capable of dissolving in and interacting with water and other polar substances. The hydrophilic polymer contained in the inventive compound carries a bile acid or a derivative thereof, and a hydroxyapatite-directed substance having a photodegradable protective group. The hydrophilic polymer contained in the inventive compound can be of any type. The molecular weight of the hydrophilic polymer is not particularly limited, and can be, for example, several thousand to several hundred thousand. Nor the structure of the hydrophilic polymer is particularly limited, and can be linear, branched, cyclic, or of the like. A preferred hydrophilic polymer is one which has low or no cytotoxicity, or one which has high biocompatibility.

Examples of hydrophilic polymers include polyethylene glycol, polyvinyl alcohol, poly(vinylpyrrolidone), polyvinylamide, polyacrylic acid, polyacrylamide, polyamino acids, polysaccharides, and proteins. Examples of preferred hydrophilic polymers include polyethylene glycol (PEG), polyvinyl alcohol, polyglutamic acid, polyaspartic acid, polylysine, and polyhistidine. More preferred examples of the hydrophilic polymers include PEG and polyvinyl alcohol. Even more preferred examples of the hydrophilic polymers include PEG. It goes without saying that the hydrophilic polymers contained in the inventive compound are not limited to the above examples.

When the hydrophilic polymer is PEG, it can be any type of PEG. The form of PEG is not particularly limited, and linear, Y-shaped, branched, etc. can be used. Preferably, branched PEGs are used in the inventive compound. Highly branched PEGs are preferred. Four-arm and eight-arm PEGs are commercially available and can be used. The molecular weight of PEG is not particularly limited, and PEG can have a molecular weight of several thousand to several hundred thousand, for example, about 5,000 to about 200,000, about 10,000 to about 100,000, or about 20,000 to about 60,000.

Derivatives of hydrophilic polymers can be appropriately produced by those skilled in the art, and are also commercially available. The functional groups in the hydrophilic polymer can be amidated, esterified, alkylated, halogenated, or converted similarly by known methods. For example, an amino group, a maleimide group, a carboxyl group, or the like can be bonded to the end of polyethylene glycol by known methods. Derivatives of the hydrophilic polymers can be selected depending on the bile acid or a derivative thereof to be bonded to the hydrophilic polymer, and on the type and structure of the hydroxyapatite-directed substance having a photodegradable protective group.

Bile acid is a steroid compound which is biosynthesized from cholesterol in the mammalian liver. Bile acid contributes to the adsorption of the inventive compound on cells. A variety of bile acids is known, and an appropriate one can be selected and used in the inventive compound. Bile acids include primary bile acids such as cholic acid and chenodeoxycholic acid; secondary bile acids such as deoxycholic acid, lithocholic acid, ursodeoxycholic acid, and hyodeoxycholic acid; and conjugated bile acids such as glycocholic acid and taurocholic acid; any of which can be used in the inventive compound. Preferably, deoxycholic acid or cholic acid is used. More preferably, deoxycholic acid (DCA) is used.

Derivatives of bile acids are known, can be appropriately synthesized, and are also commercially available. For example, any hydroxyl group of the bile acid can be attached or detached, or can be alkylated or converted similarly. Also, for example, the carboxyl groups of bile acids can be esterified. The derivatives of bile acids are not limited to those mentioned above.

The hydroxyapatite-directed substance having photodegradable protective group is a hydroxyapatite-directed substance to which a photodegradable protective group is bonded. The bonding type is not particularly limited, but is generally a chemical bond, and generally a covalent bond. By generating a covalent bond between the functional group in the hydroxyapatite-directed substance and the functional group in the photodegradable protective group, it is possible to bond the hydroxyapatite-directed substance with the photodegradable protective group. For example, if a hydroxyapatite-directed substance has a phosphate group, a photodegradable protective group can be covalently bonded to the hydroxyapatite-directed substance through the phosphate group.

Hydroxyapatite-directed substances are substances which have a high affinity or specific binding ability for hydroxyapatite, and any substance with such properties can be suitable. Hydroxyapatite-directed substances impart to cells the directionality to hydroxyapatites. Various types of hydroxyapatite-directed substances are known. Hydroxyapatite-directed substances can be naturally occurring or artificially prepared. Examples of hydroxyapatite-directed substances include, but are not limited to, bisphosphonates, tetracycline, and hydroxyapatite-binding peptides (such as ESQES sequence and CMLPHHGAC sequence). Preferred hydroxyapatite-directed substances include, for example, those with phosphate groups such as bisphosphonates. Bisphosphonates are known, and the examples include, but are not limited to, etidronic acid, clodronic acid, tiludronic acid, pamidronic acid, neridronic acid, olpadronic acid, alendronic acid, ibandronic acid, tiludronic acid, incadronic acid, risedronic acid, minodronic acid, and zoledronic acid. Even if hydroxyapatite-directed substances have cytotoxicity, bonding them to hydrophilic polymers or derivatives thereof can reduce cellular uptake, potentially alleviating their toxicity.

Photodegradable protective groups are protective groups which can be removed by light irradiation, and various types are known. Examples of photodegradable protective groups include, but are not limited to, 7-(diethylamino)coumarin, 7-(diethylamino)-4-(hydroxymethyl)coumarin, 6-bromo-7-hydroxy-4-(hydroxymethyl)coumarin, 4-(bromomethyl)-7-(diethylamino)coumarin, 1-(2-nitrophenyl)ethanol, 2-(2-nitrophenyl)propan-1-ol, 4',5'-dimethoxy-2'-nitroacetophenone, 1-(6-nitro-1,3-benzodioxole-5-yl)ethanol, 2-nitrobenzyl derivatives, allyl methyl derivatives, allyl carbonyl methyl derivatives, BODIPY derivatives, and Ru complex derivatives. The type of light used for irradiation is not particularly limited, but it is generally visible light.

The inventive compounds can be synthesized by bonding a hydrophilic polymer or a derivative thereof, a bile acid or a derivative thereof, and a hydroxyapatite-directed substance. The bonding type is not particularly limited, but is generally a covalent bond. In synthesizing the inventive compound, a photodegradable protective group can be provided after a hydroxyapatite-directed substance is bonded to a hydrophilic polymer or a derivative thereof, or a hydroxyapatite-directed substance already bonded to a photodegradable protective group can be bonded to a hydrophilic polymer or a derivative thereof. The preferred ratio (molar ratio) of the hydroxyapatite-directed substance to be bonded to the hydrophilic polymer or a derivative thereof and the bile acid or a derivative thereof varies depending on the structure and properties of the hydrophilic polymer or a derivative thereof, the hydroxyapatite-directed substance, and the bile acid or a derivative thereof, as well as on the type of cells on which the inventive compound is adsorbed, and can be determined by those skilled in the art through simple experiments. Typically, when the above ratio is 1:1 or close to it, for example, about 2:8 or about 8:2, about 3:7 or about 7:3, about 4:6 or about 6:4, the number of cells adsorbed on hydroxyapatite tends to increase, but this is not limited to these ratios.

Functional groups in hydrophilic polymers or derivatives thereof, functional groups in bile acids or derivatives thereof, and functional groups in hydroxyapatite-directed substances can be used for bonding. Bile acids or derivatives thereof, as well as hydroxyapatite-directed substances, can be bonded to any position of, but are preferably bonded near or at the terminal of hydrophilic polymers or derivatives thereof. The bonding type can be appropriately selected by those skilled in the art. For example, bile acids or derivatives thereof, as well as hydroxyapatite-directed substances, can be bonded to hydrophilic polymers by known techniques such as amide formation, ester formation, nucleophilic substitution reaction (e.g., SN₂ reaction using a tosyl group), and click reaction. For example, PEG having an amino group at its terminal end can be used, and the amino group can be condensed with a carboxyl group of deoxycholic acid using a condensing agent such as DMT-MM to form an amide (peptide bond), thereby bonding PEG to deoxycholic acid. In addition, for example, PEG having an amino group or carboxyl group at its terminal end and hydroxyapatite-directed substance having a carboxyl group or amino group at its terminal end can be used to form a peptide bond in the same manner as described above, thereby bonding the PEG to the hydroxyapatite-directed substance. Alternatively, for example, PEG having a carboxyl group at its terminal end can be used, and an ester bond can be formed between the carboxyl group and the hydroxyl group of the hydroxyapatite-directed substance, thereby bonding the PEG to the hydroxyapatite-directed substance. The bonding of a hydrophilic polymer or a derivative thereof, a bile acid or a derivative thereof, and a hydroxyapatite-directed substance is not limited to the above examples. PEG having a functional group attached to its terminal end can be synthesized using known methods and materials. PEG having a functional group attached to its terminal end is commercially available, and such PEG can also be used. The proportion between the hydrophilic polymer or a derivative thereof, the bile acid or a derivative thereof, and the hydroxyapatite-directed substance can also be appropriately determined.

The hydrophilic polymer contained in the compound of the present invention can be one type, or two or more types. The bile acid contained in the inventive compound can be one type, or two or more types. The hydroxyapatite-directed substance contained in the inventive compound can be one type, or two or more types. The photodegradable protective group contained in the present invention can be one type, or two or more types. The inventive compound can include functional molecules such as molecules which promote tissue-specific bonding, therapeutic agents, and labels.

The inventive compound imparts to cells the directionality to hydroxyapatites in response to light irradiation. Hydroxyapatite is abundant in bones and teeth. Moreover, hydroxyapatite is also present in many ossified or calcified tissues. Therefore, the cells adsorbed with the inventive compound can respond to light irradiation and approach or attach to hydroxyapatite-containing tissues such as bone, teeth, or other ossified or calcified tissues.

In another aspect, the present invention provides a method for producing cells directing to hydroxyapatites in response to light irradiation, comprising adsorbing an inventive compound on cells.

Cells to adsorb the inventive compound can be of any type. For example, mesenchymal stem cells (MSC), induced pluripotent stem cells (iPS cells), osteoprogenitor cells, and osteoblasts, which all have adsorbed the inventive compound, can be applied to sites requiring an augmentation or regeneration of bone, such as those in cases of bone defects due to trauma or osteoporosis. Also, for example, mesenchymal stem cells (MSC), induced pluripotent stem cells (iPS cells), osteoclasts, and osteoclast precursor cells, which have adsorbed the inventive compound can be applied to sites requiring suppression or treatment of ossification or calcification, such as heterotopic ossification sites and calcified sites (for example, calcification sites of stents in the treatment of atherosclerosis).

The inventive compound can be adsorbed on cells by contacting the inventive compound with cells. For example, the inventive compound can be adsorbed on cells by incubating the compound and cells in a medium. Preferably, the medium is an aqueous medium, which can be a cell culture medium. Media for cell culture are known and can be appropriately selected depending on the type of cells. Conditions such as temperature and time for incubation can also be appropriately determined by those skilled in the art. For example, the cells can be incubated in DMEM medium at 37°C for 12 to 36 hours. The ratio of the inventive compound to cells (the concentration of the inventive compound in the medium) can also be appropriately determined by those skilled in the art. For example, the inventive compound can be incubated at a concentration of several µM to several tens of µM with respect to 10⁴ to 10⁸ cells/mL. As mentioned above, the cells adsorbed with the inventive compound can be obtained with a simple procedure of incubating the cells with the inventive compound. That is, according to the present invention, functional cells can be obtained without requiring complex operations such as gene manipulation.

In a further aspect, the present invention provides an agent for imparting to cells the directionality to hydroxyapatites in response to light irradiation, comprising the inventive compound. The dosage form of the above agent can be solid (powder, granules, etc.), liquid (solution, suspension, etc.), or semi-solid (paste, etc.).

In a further aspect, the present invention provides a kit for imparting to cells the directionality to hydroxyapatites in response to light irradiation, comprising the inventive compound. The above kit contains the inventive compound as a component. For example, the above agent can be included in the package of the above kit. Typically, the kit will come with an instruction manual.

In a further aspect, the present invention provides cells on which the inventive compound has been adsorbed. The cells adsorbed with the inventive compound can be applied to the disease site using known administration methods and means. There are no specific limitations on the method of administration. The disease site can be any site containing hydroxyapatite, and is not particularly limited. The disease site can be a region where bone loss or reduced bone mass has occurred due to, for example, trauma or disease, and can also be a calcified stent. The cells adsorbed with the inventive compound can also be administered to the disease site by direct application. The cells adsorbed with the inventive compound can also be administered to the disease site by injection. The cells adsorbed with the inventive compound can be administered to the site exposed by surgery.

Light can be irradiated using known means and methods. If the disease site is on the surface of the body, direct light irradiation can be used. If the disease site is at a shallow position and light can penetrate to the site through the skin, light can be irradiated on the skin. If the disease site is located deep and light cannot penetrate to the site through the skin, light can be irradiated using endoscopes, catheters, optical fibers and the like. Light irradiation makes it possible to deliver the cells adsorbed with the inventive compound to a desired disease site at a desired timing.

In a further aspect, the present invention provides pharmaceutical compositions for the treatment of diseases which require an augmentation or regeneration of bone, or for the treatment of ossification or calcification, which contains the cells adsorbed with the inventive compound. The pharmaceutical composition of the present invention can also be used, for example, for the treatment of trauma or osteoporosis. The pharmaceutical composition of the present invention can also be used, for example, for the treatment of heterotopic ossification or stent calcification. The pharmaceutical composition of the present invention can also be used, for example, in dental treatments.

In a further aspect, the present invention provides a method for treating diseases which require an augmentation or regeneration of bone, or a method for treating ossification or calcification, comprising administering cells adsorbed with the inventive compound to the disease site.

In a further aspect, the present invention provides the use of cells adsorbed with the inventive compound, for the method of augmentation of bone or the production of pharmaceutical composition for the treatment of diseases requiring bone regeneration, the ossification, or calcification.

In a further aspect, the present invention provides cells adsorbed with the inventive compound, which are used for the method of augmentation of bone or the treatment of diseases requiring bone regeneration, the ossification, or calcification.

In a further aspect, the present invention provides a compound comprising a hydrophilic polymer or a derivative thereof, a bile acid or a derivative thereof, and a hydroxyapatite-directed substance.

As used herein, the terms shall be construed as having the meanings generally understood by those skilled in the art, unless otherwise specified. As used herein, a value with the notation 'about' is understood to have a range of ±20% of that value, preferably ±15%, and even more preferably ±10%.

### Examples

Hereinafter, the present invention is described in more detail and specifically with reference to examples, but the scope of the present invention is not limited to these examples.

### Example 1

The bondings of 8PEG40K-ALN50-DCA50-FGA and 4PEG20K-ALN50-DCA50-FGA to hydroxyapatite were investigated.

8PEG40K-ALN50-DCA50-FGA is a compound in which deoxycholic acid (DCA) and alendronic acid (ALN) are introduced at a ratio of 50:50 at the chain ends of an 8-branched PEG with a molecular weight of 40,000. 4PEG20K-ALN50-DCA50-FGA is a compound in which deoxycholic acid (DCA) and alendronic acid (ALN) are introduced at a ratio of 50:50 at the chain ends of a 4-branched PEG with a molecular weight of 20,000.

### (1) Synthesis of test compounds

Alendronic acid was condensed with 8PEG40K or 4PEG20K bearing a terminal carboxylic acid activated by NHS ester. Then, 8PEG40K-ALN50-DCA50 and 4PEG20K-ALN50-DCA50 were obtained by using a condensing agent (DMT-MM) to condense ethylenediamine-DCA. Then, for the two polymers, 8PEG40K-ALN50-DCA50-FGA and 4PEG20K-ALN50-DCA50-FGA were obtained by using DMT-MM to condense fluorescent substance (FGA, fluorescein glycine amide).

### (2) The bondings of 8PEG40K-ALN50-DCA50-FGA and 4PEG20K-ALN50-DCA50-FGA to hydroxyapatite

8PEG40K-ALN50-DCA50-FGA or 4PEG20K-ALN50-DCA50-FGA was mixed with hydroxyapatite granules (10 mg) in HEPES buffer and left to stand at 37°C for 30 minutes. The amount of hydroxyapatite non-adsorptive polymer was removed from the mixed solution by ultrafiltration (pore size 0.45 µm) and washing, and the resulting hydroxyapatite-polymer complexes were observed using a fluorescence microscope (Figure 1). In addition, the amount of hydroxyapatite non-adsorptive polymer in the filtrate was quantified from the fluorescence intensity (Figure 2).

It was confirmed that 8PEG40K-ALN50-DCA50-FGA and 4PEG20K-ALN50-DCA50-FGA adsorbed on hydroxyapatite (Figure 1, bright areas; a similar image was obtained for 4PEG20K-ALN50-DCA50-FGA as well). The adsorption amount increased depending on the concentration (Figure 2).

### Example 2

The adsorption of 8PEG40K-ALN50-DCA50-FGA and 4PEG20K-ALN50-DCA50-FGA to cells was investigated.

### Synthesis of test compounds

Alendronic acid was condensed with 8PEG40K or 4PEG20K bearing a terminal carboxylic acid activated by NHS ester. Then, 8PEG40K-ALN50-DCA50 and 4PEG20K-ALN50-DCA50 were obtained by using a condensing agent (DMT-MM) to condense ethylenediamine-DCA. Then, for the two polymers, 8PEG40K-ALN50-DCA50-FGA and 4PEG20K-ALN50-DCA50-FGA were obtained by using DMT-MM to condense fluorescent substance (FGA).

### (2) Adsorption test on normal human skin fibroblasts

8PEG40K-ALN50-DCA50-FGA or 4PEG20K-ALN50-DCA50-FGA was mixed with normal human skin fibroblasts in HEPES buffer and left to stand at 37°C for 30 minutes. After removing the cell non-adsorptive polymer by centrifugation and washing, the cell-polymer complexes were observed using a fluorescence microscope (Figure 3).

As shown in Figure 3, green fluorescence was observed on the cells, confirming that 8PEG40K-ALN50-DCA50-FGA and 4PEG20K-ALN50-DCA50-FGA were adsorbed on the cells (cells appear bright).

It was also confirmed that the adsorption of 8PEG40K-ALN50-DCA50-FGA and 4PEG20K-ALN50-DCA50-FGA did not affect cell proliferation.

### Example 3

The photodeprotection reaction of 4PEG20K-ALN100-DEACM (alendronic acid protected with 7-(diethylamino)coumarin (DEACM)) (protective group introduction rate: 50%) was investigated.

### (1) Synthesis of test compounds

Alendronic acid was condensed with 4PEG20K, followed by condensation with 7-(diethylamino)coumarin bromide in the presence of 4 equivalents of tertiary butylammonium hydroxide (TBA-OH) to obtain 4PEG20K-ALN100-DEACM.

### (2) Deprotection of photoprotective groups

One mg of 4PEG20K-ALN100-DEACM was dissolved in MilliQ and then irradiated with light for 60 seconds (irradiation light wavelength: 405 nm, intensity: 50 mW/cm²). The solution after light irradiation was diluted with DMSO (MilliQ(vol%): DMSO (vol%) = 50:50), and 4PEG20K-ALN100-DEACM was removed by ultrafiltration (molecular weight cutoff: 3,000). The deprotection rate was quantified from the absorbance (390 nm) derived from the deprotected DEACM group in the filtrate. When the 4PEG20K-ALN100-DEACM was subjected to ultrafiltration before light irradiation, no UV absorption derived from DEACM was observed, whereas after light irradiation, a 75% release of DEACM-based groups (i.e., deprotection) was observed (Figure 4).

### Example 4

Mesenchymal stem cells were adsorbed with 4PEG20K-ALN50-DCA50-FGA and were investigated whether the cells were accumulated in mouse bone.

4PEG20K-ALN50-DCA50-FGA was mixed with mesenchymal stem cells in HEPES buffer, and left to stand for 30 minutes at 37°C under 5% CO₂ atmosphere. After removing the cell non-adsorptive polymer by centrifugation and washing, the polymer-attached (modified) cells were adhered to the mouse bone. The cells were stained with CellTracker Green (Figure 5).

As shown in the left panel of Figure 5, green fluorescence was observed on the mouse bone (the bright areas on the cells). The results of quantifying the fluorescence intensity on the bone are shown in Figure 6. From these results, it was confirmed that cells adhered with 4PEG20K-ALN50-DCA50-FGA were accumulated in mouse bone.

### Example 5

8PEG40k-ALN50-DCA50-FGA was adsorbed on osteoclast precursor cells.

### (1) Adsorption of 8PEG40k-ALN50-DCA50-FGA on osteoclast precursor cells

8PEG40K-ALN50-DCA50-FGA was obtained in the same manner as in Example 2. In 500 µL of αMEM medium was dissolved 12.5 µM of the above compound, then was added 1.0 × 10⁶ of osteoclast precursor cells, which was cultured for a specified time. The fluorescence intensity of the supernatant was measured every hour to calculate the amount of inserted polymer. As shown in the left panel of Figure 7, the adsorption amount of 8PEG40k-ALN50-DCA50-FGA on osteoclast precursor cells increased over time, reaching a maximum after 8 hours. The right panel of Figure 7 shows a confocal fluorescence microscopic image depicting the adsorption of 8PEG40k-ALN50-DCA50-FGA on osteoclast precursor cells. Fluorescence of FGA was observed around and on the surface of the cells.

8PEG40k-ALN50-DCA50-FGA adsorbed on osteoclast precursor cells were also investigated for its support by the cell membrane. It was confirmed that approximately 40% of 8PEG40k-ALN50-DCA50-FGA was retained after 24 hours.

Furthermore, the proliferation of osteoclast precursor cells adsorbed with 8PEG40k-ALN50-DCA50-FGA was also investigated. It was confirmed that the adsorption of 8PEG40k-ALN50-DCA50-FGA did not affect cell proliferation.

### (2) The hydroxyapatite recognition ability of osteoclast precursor cells adsorbed with 8PEG40k-ALN50-DCA50-FGA.

Osteoclast precursor cells adsorbed with 8PEG40k-ALN50-DCA50-FGA were obtained by using the same procedure as above (1). The obtained cell suspension was added to an Eppendorf tube containing 5 mg of hydroxyapatite deposit, was then incubated for 30 minutes, and after incubation, was washed and observed using a confocal laser microscopy.

The upper panels in the left of Figure 8 show the results of adding 8PEG40k-ALN50-DCA50-FGA-adsorbed osteoclast precursor cells to hydroxyapatite, while the lower panels show the results of adding non-adsorbed cells to hydroxyapatite. Fluorescence was observed around the hydroxyapatite (which appears black), confirming that osteoclast precursor cells adsorbed with 8PEG40k-ALN50-DCA50-FGA adsorbed on hydroxyapatite (green fluorescence: 8PEG40k-ALN50-DCA50-FGA, blue fluorescence: cell nucleus). The right panel of Figure 8 shows the results of analyzing and quantifying the blue fluorescent intensity of the microscopic image using ImageJ. The fluorescence of osteoclast precursor cells adsorbed with 8PEG40k-ALN50-DCA50-FGA was 8.8 times that of non-adsorbed cells. The above results indicate that the adsorption of 8PEG40k-ALN50-DCA50-FGA improved the cell's hydroxyapatite affinity.

It was also confirmed that osteoclast precursor cells adsorbed with 8PEG40k-ALN50-DCA50-FGA proliferated on plates coated with hydroxyapatite, similar to non-adsorbed cells.

### (3) The osteolytic action of osteoclast precursor cells adsorbed with 8PEG40k-ALN50-DCA50-FGA.

Osteoclast precursor cells adsorbed with 8PEG40k-ALN50-DCA50-FGA were dispersed on plates coated with hydroxyapatite (1.0 x 10⁴ cells/well), incubated at 37°C for 1 hour, washed. After further incubation at 37°C for 10 days, the osteolytic action was investigated using a bone resorption activity assessment kit (Iwai Chemical Co., Ltd.). The osteolytic action of osteoclasts adsorbed with 8PEG40k-ALN50-DCA50-FGA was also investigated by using the same procedure.

Figure 9 shows the results. It was confirmed that both osteoclast precursor cells (pre-OC) and osteoclasts (OC) promoted the osteolytic action by the adsorption (modification) of 8PEG40k-ALN50-DCA50-FGA.

### Example 6

In the same manner as in Example 5(1), 8PEG40k-ALN50-DCA50-FGA was adsorbed on osteoclasts, and the adsorption amount was measured.

As shown in the left panel of Figure 10, the adsorption amount of 8PEG40k-ALN50-DCA50-FGA on osteoclasts increased over time. The right panel of Figure 10 shows a confocal fluorescence microscopic image depicting the adsorption of 8PEG40k-ALN50-DCA50-FGA on osteoclasts after a 9-hour incubation. Fluorescence of FGA and fluorescence of cell nucleus were observed around and on the surface of the cells, and the adsorption of 8PEG40k-ALN50-DCA50-FGA on the cells was confirmed.

### Example 7

The inventive compound (8PEG40k-ALN50-DEACM-DCA50) introduced with a light-responsive protective group was synthesized. In this compound, the introduction rate of DEACM to alendronic acid was 100%. An overview of the synthesis procedure is shown below.

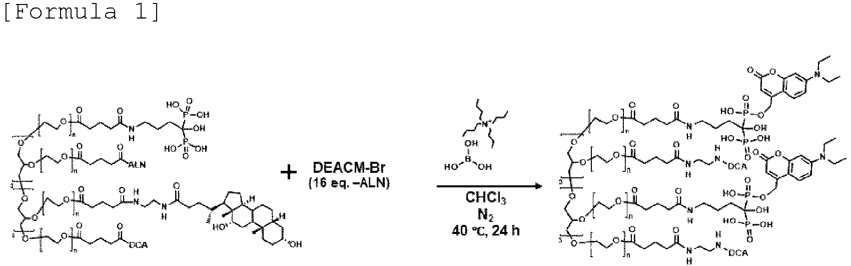

Using the method described by C. Chevalier et al. in STAR Protocols 2021, 2, 100452, osteoclast precursor cells were differentiated into osteoclasts. In a 1.5 mL Eppendorf tube, 12.5 µM of 8PEG40k-ALN50-DEACM-DCA50 was adsorbed on osteoclasts (5 x 10⁵ cells) suspended in 500 µL of α-MEM. Adsorption was conducted by incubation at 37°C for 7 hours under 5% CO₂ atmosphere. Osteoclasts adsorbed with 8PEG40k-ALN50-DEACM-DCA50 was irradiated with light (irradiation light wavelength: 405 nm, 20 minutes). In 24-well plates coated with hydroxyapatite (containing 1 mL of α-MEM) were added osteoclasts adsorbed with 8PEG40k-ALN50-DEACM-DCA50 (3.0 x 10⁴ cells/well) under conditions with and without light irradiation, which were incubated at 37°C in 5% CO₂ atmosphere for 1 hour. After incubation, the plates were washed with α-MEM and the cells adsorbed on the hydroxyapatite were counted using trypan blue staining. Figure 11 shows the results. It was confirmed that the amount of adsorbed cells increased in response to light irradiation (more than double compared to no light irradiation).

The above results indicate that the cells adsorbed with the inventive compounds can be directed to the bone disease site in response to light irradiation.

### Industrial Applicability

The present invention is useful in such fields as agents for the regeneration and augmentation of bones and teeth, as well as treatments for ossification and calcification.

## Claims

1. A compound comprising a hydrophilic polymer or a derivative thereof, a bile acid or a derivative thereof, and a hydroxyapatite-directed substance having a photodegradable protective group.

2. The compound according to claim 1, wherein the hydrophilic polymer is selected from the group consisting of polyethylene glycol, polyvinyl alcohol, polyglutamic acid, polyaspartic acid, polylysine, and polyhistidine.

3. The compound according to claim 2, wherein the hydrophilic polymer is polyethylene glycol or polyvinyl alcohol.

4. The compound according to claim 1, wherein the bile acid is selected from the group consisting of cholic acid, chenodeoxycholic acid, deoxycholic acid, lithocholic acid, ursodeoxycholic acid, hyodeoxycholic acid, glycocholic acid, and taurocholic acid.

5. The compound according to claim 4, wherein the bile acid is deoxycholic acid or cholic acid.

6. The compound according to claim 1, wherein the hydroxyapatite-directed substance is selected from the group consisting of bisphosphonates, tetracycline, and hydroxyapatite binding peptides.

7. The compound according to claim 6, wherein the hydroxyapatite-directed substance is a bisphosphonate.

8. The compound according to claim 7, wherein the bisphosphonate is selected from the group consisting of etidronic acid, alendronic acid, risedronic acid, minodronic acid, ibandronic acid, and zoledronic acid.

9. The compound according to claim 1, wherein the photodegradable protective group is selected from the group consisting of 7-(diethylamino)coumarin, 7-(diethylamino)-4-(hydroxymethyl)coumarin, 6-bromo-7-hydroxy-4-(hydroxymethyl)coumarin, 4-(bromomethyl)-7-(diethylamino)coumarin, 1-(2-nitrophenyl)ethanol, 2-(2-nitrophenyl)propan-1-ol, 4',5'-dimethoxy-2'-nitroacetophenone, 1-(6-nitro-1,3-benzodioxole-5-yl)ethanol, 2-nitrobenzyl derivatives, allyl methyl derivatives, allyl carbonyl methyl derivatives, BODIPY derivatives, and Ru complex derivatives.

10. A method for producing cells directing to hydroxyapatite in response to light irradiation, comprising adsorbing the compound according to any one of claims 1 to 9 on cells.

11. An agent for imparting to cells directionality to hydroxyapatite in response to light irradiation, comprising the compound according to any one of claims 1 to 9.

12. A kit for imparting to cells directionality to hydroxyapatite in response to light irradiation, comprising the compound according to any one of claims 1 to 9.

13. Cells on which the compound according to any one of claims 1 to 9 is adsorbed.

14. The cells according to claim 13, wherein they are mesenchymal stem cells, induced pluripotent stem cells, osteoblasts, osteoclasts, osteoprogenitor cells, or osteoclast precursor cells.

15. A compound comprising a hydrophilic polymer or a derivative thereof, a bile acid or a derivative thereof, and a hydroxyapatite-directed substance.
